# EUROPEAN PATENT APPLICATION

(11) **EP 1 693 467 A1**
(43) Date of publication of application: **23.08.2006**
(21) Application number: 06003204.2
(22) Date of filing: 17.02.2006
(51) Int. Cl.: C12Q 1/68

(54) **Stanniocalcin (STC1) mRNA assay method**

(30) Priority: 17.02.2005 JP 2005041148
(71) Applicant: TOSOH CORPORATION, Shunan-shi Yamaguchi-ken 746-8501 (JP)
(72) Inventor: Saito, Juichi, Yamato-shi Kanagawa (JP); Hayashi, Toshinori, Sagamihara-shi Kanagawa (JP)
(74) Representative: Pautex Schneider, Nicole Véronique

(57) **Abstract**

The present invention provides an RNA amplification process, comprising steps in which double-stranded DNA containing a promoter sequence is produced using a first primer and second primer, at least one of which has the promoter sequence at the 5' end, and reverse transcriptase, the double-stranded DNA is used as template for production of RNA transcript using RNA polymerase and the RNA transcript is used as template for subsequent DNA synthesis with the reverse transcriptase to produce the double-stranded DNA. The process yields an amplified RNA product which is assayed with a fluorescent intercalator dye-labeled nucleic acid probe.

SEQUENCE LISTING

<110> TOSOH CORPORATION
<120> Stanniocalcin-1 (STC1) mRNA assay method
<130> R433
<160> 17
<170> PatentIn version 3.3

<210> 1
<211> 63
<212> DNA
<213> Artificial
<220>
<223> First primer
<400> 1
ctccacctgt gacacagatg ggatgtatga catctgtaaa tccttcttgt acagcgctgc 60
taa 63

<210> 2
<211> 87
<212> DNA
<213> Artificial
<220>
<223> Second primer
<400> 2
gcaatcatcc tttggaaagt ggagcacctc cgaatggcga ggaagacctt ggaggtgacc 60
ccgttggcga tgcattttaa gctctct 87

<210> 3
<211> 54
<212> DNA
<213> Artificial
<220>
<223> Intercalator dye-labeled nucleic acid probe
<400> 3
ttggcgatgc attttaagct ctctttgacg aatgcttttc cctgagtgtc aaat 54

<210> 4
<211> 23
<212> DNA
<213> Artificial
<220>
<223> First primer

<400> 4
ctccacctgt gacacagatg gga                                              23

<210> 5
<211> 23
<212> DNA
<213> Artificial
<220>
<223> First primer
<400> 5
gtatgacatc tgtaaatcct tct                                              23

<210> 6
<211> 24
<212> DNA
<213> Artificial
<220>
<223> First primer
<400> 6
aatccttctt gtacagcgct gcta                                             24

<210> 7
<211> 24
<212> DNA
<213> Artificial
<220>
<223> Second primer
<400> 7
ttggcgatgc attttaagct ctct                                             24

<210> 8
<211> 24
<212> DNA
<213> Artificial
<220>
<223> Second primer
<400> 8
tggcgaggaa gaccttggag gtga                                             24

<210> 9
<211> 24
<212> DNA
<213> Artificial
<220>

<223> Second primer
<400> 9
aatcatcctt tggaaagtgg agca                                              24

<210> 10
<211> 24
<212> DNA
<213> Artificial
<220>
<223> Intercalator dye-labeled nucleic acid probe
<400> 10
cgaatgcttt tccctgagtg tcaa                                              24

<210> 11
<211> 24
<212> DNA
<213> Artificial
<220>
<223> Intercalator dye-labeled nucleic acid probe
<400> 11
ttggcgatgc attttaagct ctct                                              24

<210> 12
<211> 22
<212> DNA
<213> Artificial
<220>
<223> Intercalator dye-labeled nucleic acid probe
<400> 12
ttggcgatgc attttaagct ct                                                22

<210> 13
<211> 66
<212> DNA
<213> Artificial
<220>
<223> Cleavage oligonucleotide
<400> 13
aagaaggatt tacagatgtc atacatccca tctgtgtcac aggtggagtt ttccaggcat 60
gcaaaa                                                                  66

<210> 14
<211> 24
<212> DNA

<213> Artificial
<220>
<223> Cleavage oligonucleotide
<400> 14
gtggagtttt ccaggcatgc aaaa 24

<210> 15
<211> 24
<212> DNA
<213> Artificial
<220>
<223> Cleavage oligonucleotide
<400> 15
tcatacatcc catctgtgtc acag 24

<210> 16
<211> 27
<212> DNA
<213> Artificial
<220>
<223> Cleavage oligonucleotide
<400> 16
aggatttaca gatgtcatac atcccat 27

<210> 17
<211> 28
<212> DNA
<213> Artificial
<220>
<223> T7RNA polymerase promoter sequence
<400> 17
aattctaata cgactcacta tagggaga 28

## Description

### Field of Invention

The present invention relates to a method for assaying stanniocalcin-1 mRNA in a convenient, isothermal, single-stage and rapid manner. The invention relates to the field of medicine and particularly clinical diagnosis, and is useful for providing an index for early detection of leukemia and cancer, diagnosis of microresidual lesions, therapy monitoring, prognosis and treatment protocol decision-making.

### Prior Art

Stanniocalcin (hereinafter, "STC") is a glycoprotein secreted from organs unique to fish known as Stannius corpuscles, which are attached to fish kidneys, and it has been identified as a hormone that acts in the gills to regulate blood calcium levels. In recent years, cDNA has been cloned for human STC which is highly homologous with fish STC (see Chang, A. C-M. et al, (1995) Mol. Cell. Endocrinol., 112,241-247 and Olsen, H. S. et al, (1996) Procedure. Natural. Acad. Sci. USA, 93, 1792-1796). Human STC has been designated as STC1 for distinction from the later cloned STC2. STC1 has a calcium-regulating function and is believed to play a role in cellular differentiation and proliferation through calcium control. STC1 has already been correlated with a number of diseases. For example, (1) a possible connection has been found with hereditary conditions such as Huntington's disease and Werner syndrome (see Chang, A. C-M. et al, (1998) Genomics, 47, 393-398), (2) a link with osteogenesis has been suggested (see Worthington, R. A. et al, (1999) Electrophoresis, 20, 2071-2076), (3) reports show accelerated transcription of STC1 messenger RNA (mRNA) in cerebral infarction, suggesting a role in protecting neurons under ischemic or hypoxic conditions (see Japanese Patent Public Inspection No. 2003-531107; Zhang, K-Z. et al, (2000) Procedure. Natural. Acad. Sci. USA, 97, 3637-3642), and (4) the possibility of a link with atherosclerosis has also been suggested (see Sato, N. et al, (1998) J. Biochem., 123, 1119-1126). It is expected that assay of STC1 mRNA expression levels can be effective for diagnosis and observation of the course of these implicated conditions, and increase in STC1 mRNA expression levels has been noted in recent years particularly in chronic leukemia, breast cancer and other cancers, suggesting that assay of STC1 mRNA may be effective as a marker for early detection of leukemia and other cancers, as well as detection of their metastasis and microresidual lesions (Japanese Unexamined Patent Publication No. 2000-2709; Fujiwara, Y. et al, (2000) Int. J. Oncol., 16, 799-804; and Wascher, R. A. et al, (2003) Clin. Cancer Research., 9, 1427-1435). A convenient, rapid, highly sensitive and highly accurate assay method for STC1 mRNA has been desired for application toward diagnosis of such diseases.

One means for highly sensitive assay of STC1 mRNA is a method in which STC1 mRNA is amplified by RT-PCR and the amplification product is assayed, but this generally requires a two-stage process comprising a reverse transcription (RT) stage and a PCR stage, which not only complicates the procedure and leads to poor reproducibility, but also increases the risk of secondary contamination. Combining the RT stage and PCR stage also requires a period of 2 hours or longer as a rule, and therefore the method has been poorly suitable for treatment of multiple samples or for reducing examination costs. Moreover, since DNA is also amplified in RT-PCR, amplification of the mRNA alone requires complete removal of the chromosomal DNA by DNase treatment in the nucleic acid extraction step, which is thus rendered more complex. In addition, the reaction temperature must be abruptly increased for PCR, and this has constituted an obstacle against achieving energy and cost reduction for automated reaction apparatuses.

A method of quantifying target RNA that is commonly employed is Kinetic RT-PCR, wherein the PCR stage is carried out in the presence of a fluorescent intercalator dye and the increase in fluorescence is measured, but concomitant detection of non-specific amplification products such as primer dimer is a drawback in this method.

On the other hand, methods of amplifying RNA at constant temperature have been reported, such as the NASBA method (see Japanese Patent No. 2650159 and Japanese Patent No. 3152927) and the TMA method (see Japanese Patent No. 3241717). These RNA amplification methods comprise the steps of synthesis of double-stranded DNA containing a promoter sequence, using a primer comprising said promoter sequence for the target RNA, reverse transcriptase and if necessary Ribonuclease H (RNaseH), synthesis of RNA including the specific base sequence of the target RNA using RNA polymerase, and a chain reaction with the RNA as the template for synthesis of double-stranded DNA containing the promoter sequence. Amplification of the RNA is followed by detection of the amplified RNA by electrophoresis or a hybridization method employing a nucleic acid probe bound to a detectable label.

The RNA amplification methods mentioned above are suitable as convenient mRNA assay methods because they allow amplification of RNA alone at constant temperature in a single stage, but they require complex procedures for detection by hybridization methods and the like and do not allow reproducible quantification. Also, since the NASBA and TMA methods include an initial step of heating to higher temperature than the reaction temperature in order to denature the higher-order structure of the target RNA, the RNA amplification reaction is not completely isothermal from start to finish. Consequently, complexity of the reaction apparatus and an increasing number of steps have been obstacles to rapid detection. The method of Ishiguro et al. (see Japanese Unexamined Patent Publication No. 2000-14400 and Ishiguro, T. et al, (2003) Anal. Biochem., 314, 77-86) is a method for conveniently accomplishing amplification and assay of mRNA in a completely isothermal manner. This method involves first carrying out the aforementioned RNA amplification method in the presence of a nucleic acid probe which is labeled with a fluorescent intercalator dye and designed so that formation of a complementary double-stranded chain with the target nucleic acid causes the fluorescent intercalator dye portion to intercalate in the complementary double-stranded portion producing a change in the fluorescent property, and then measuring the change in the fluorescent property. This method allows RNA amplification and assay to be carried out simultaneously in a convenient, isothermal and single-stage manner, in a closed vessel. However, the fact that the method of Japanese Unexamined Patent Publication No. 2000-14400 is carried out in a completely isothermal manner is considered to be the reason for formation of a higher-order structure by the mRNA being measured, which inhibits oligonucleotide binding. Thus, convenient and rapid assay of STC1 mRNA requires an oligonucleotide sequence which allows amplification and detection of the STC1 mRNA without reduced binding efficiency even at constant temperature.

### Disclosure of Invention

Assay of stanniocalcin-1 mRNA is useful for early detection of leukemia, breast cancer and other cancers, as well as detection of their microresidual lesions and monitoring of their progression, but is problematic in that RT-PCR involves a two-stage process and the procedure is complex and requires abrupt increase in the reaction temperature, which are problems that have led to the risk of secondary contamination and poor reproducibility, while also constituting an obstacle to convenient assay and automation. The present invention overcomes these problems by providing a method for assaying stanniocalcin-1 mRNA conveniently, rapidly, isothermally and in a single stage.

As a result of much diligent research directed toward solving the aforementioned problems, the present inventors have developed a convenient, rapid, isothermal and single-stage assay method for stanniocalcin-1 mRNA which makes use of the aforementioned RNA amplification method. Specifically, we have realized convenient, isothermal, single-stage assay of stanniocalcin-1 mRNA, by a method of measuring the amount of RNA product amplified in an RNA amplification step that comprises producing double-stranded DNA which contains a promoter sequence using a first primer and a second primer (either or both of which has the promoter sequence at the 5' end), producing RNA transcript using the double-stranded DNA as template, and using the RNA transcript as template for subsequent DNA synthesis to produce the double-stranded DNA. In particular, the present application includes the following inventions:
[1] A method for assaying stanniocalcin-1 mRNA present in a sample, which method comprises
   (1) a step of producing double-stranded DNA which contains a promoter sequence and a specific base sequence within said RNA downstream from said promoter sequence, using a first primer which is homologous to at least one section downstream from the 5' end of said specific base sequence and a second primer which is complementary to at least one section upstream from the 3' end of said specific base sequence, wherein said promoter sequence is at the 5' end of either or both said first and second primers,
   (2) a step of producing RNA transcript using said double-stranded DNA as template,
   (3) a step of amplifying said RNA transcript in a chain-reaction manner using said RNA transcript as template for subsequent DNA synthesis, and
   (4) a step of measuring the amount of said RNA transcript.
[2] A method for assaying stanniocalcin-1 mRNA of [1], characterized by using a combination of said first and second primers, wherein said first primer comprises at least 15 contiguous bases of the sequence listed as SEQ ID NO: 1, and said second primer comprises at least 15 contiguous bases of the sequence listed as SEQ ID NO: 2.
[3] A method for assaying stanniocalcin-1 mRNA of [1] or [2], characterized in that the assay of the amount of said RNA transcript is carried out by measuring the change in the fluorescent property of a nucleic acid probe which is labeled with a fluorescent intercalator dye and designed so that formation of a complementary double-stranded chain with the target nucleic acid causes the fluorescent intercalator dye portion to intercalate in said complementary double-stranded portion producing a change in its fluorescent property.
[4] The method for assaying stanniocalcin-1 mRNA of [3], characterized by using a combination of oligonucleotides consisting of said first primer comprising at least 15 contiguous bases of the sequence listed as SEQ ID NO: 1, said second primer comprising at least 15 contiguous bases of the sequence listed as SEQ ID NO: 2, and said fluorescent intercalator dye-labeled nucleic acid probe comprising at least 15 contiguous bases of the sequence listed as SEQ ID NO: 3 or the sequence complementary to said sequence.
[5] An assay reagent for stanniocalcin-1 mRNA, characterized by containing as constituent elements oligonucleotides which have a combination of sequences consisting of a first primer comprising at least 15 contiguous bases of the sequence listed as SEQ ID NO: 1, and a second primer comprising at least 15 contiguous bases of the sequence listed as SEQ ID NO: 2, wherein a promoter sequence is at the 5' end of either or both of said first and second primers.
[6] An assay reagent for stanniocalcin-1 mRNA, characterized by containing as constituent elements oligonucleotides which are a combination of sequences consisting of a first primer comprising at least 15 contiguous bases of the sequence listed as SEQ ID NO: 1, a second primer comprising at least 15 contiguous bases of the sequence listed as SEQ ID NO: 2, wherein a promoter sequence is at the 5' end of either or both of said first and second primers, and a fluorescent intercalator dye-labeled nucleic acid probe comprising at least 15 contiguous bases of the sequence listed as SEQ ID NO: 3 or the sequence complementary to said sequence.
[7] Stanniocalcin-1 mRNA or a specific oligonucleotide which is complementary thereto, characterized by comprising at least 15 contiguous bases of the sequence listed as any one of SEQ ID NO: 1 to 3, or the sequence complementary to said sequence.

### BRIEF DESCRIPTION OF THE DRAWINGS

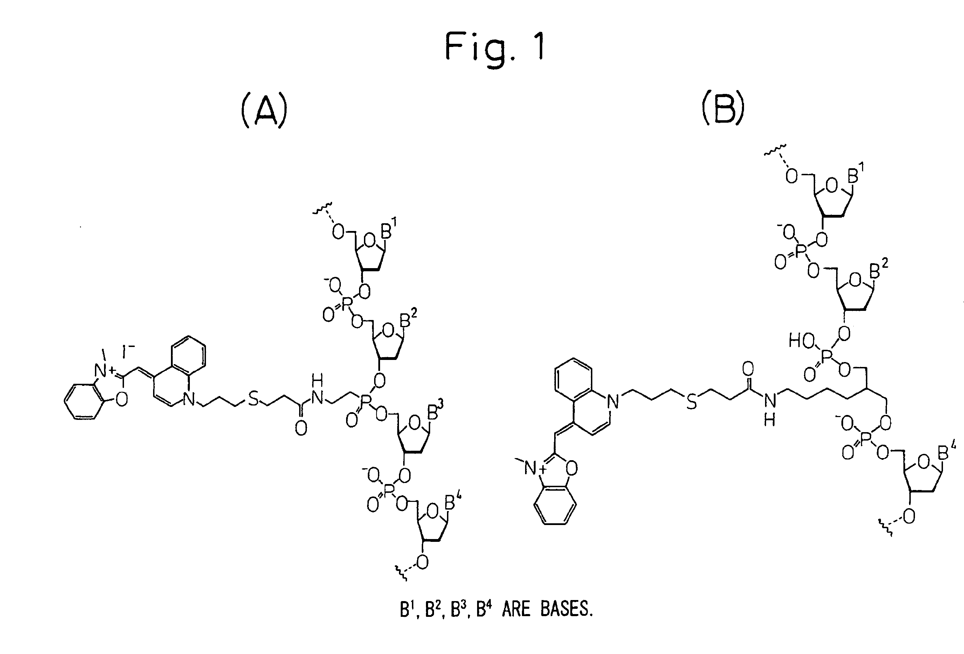

Fig. 1 shows the structure of the fluorescent intercalator dye-labeled nucleic acid probe constructed in Example 2. B¹, B², B³ and B⁴ represent bases. A) Probe obtained by bonding a fluorescent intercalator dye (oxazole yellow) via a linker at the phosphate diester portion, according to the method of Ishiguro et al. (1996). The 3' end-OH was modified with glycolic acid to prevent extension reaction from the 3' end-OH. B) Probe obtained by using commercially available Label-ON Reagents (Clontech) for introduction of an amino group, and bonding oxazole yellow according to the method of Ishiguro et al. (Ishiguro et al (1996)). In this case, the nucleoside portion of the introduction site (B³ in the drawing) was eliminated and the amino group introduced therein. The 3' end-OH was modified with biotin to prevent extension reaction from the 3' end-OH.

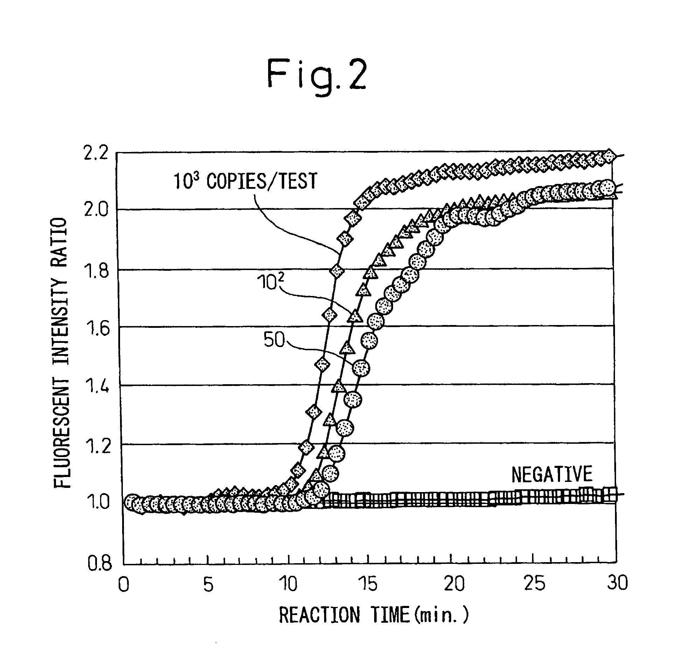

Fig. 2 shows the fluorescence profile obtained from the measurement results of Example 3. The results are from time periodic measurement of the fluorescent intensity (excitation wavelength: 470 nm, fluorescent wavelength: 520 nm) simultaneously with RNA amplification according to the invention. The horizontal axis represents reaction time and the vertical axis represents fluorescent intensity ratio (fluorescent intensity of reaction mixture/background fluorescence). Each number of copies shown in the graph represents the initial number of copies of the STC1 RNA (containing bases 242-1028) used in each test (calculated from absorbance at 260 nm) .

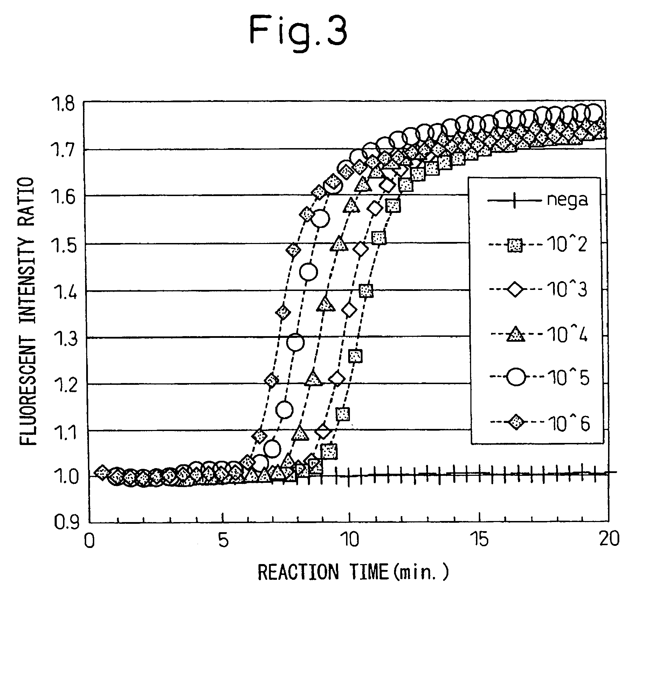

Fig. 3 shows the fluorescence profile obtained from the measurement results of Example 4. The results are from time periodic measurement of the fluorescent intensity (excitation wavelength: 470 nm, fluorescent wavelength: 520 nm) simultaneously with RNA amplification according to the invention. The horizontal axis represents reaction time and the vertical axis represents fluorescent intensity ratio (fluorescent intensity of reaction mixture/background fluorescence). The values in the legend for the graph represent the initial numbers of copies of STC1 RNA used for each test (calculated from absorbance at 260 nm). Also, "nega" represents the negative control (diluent).

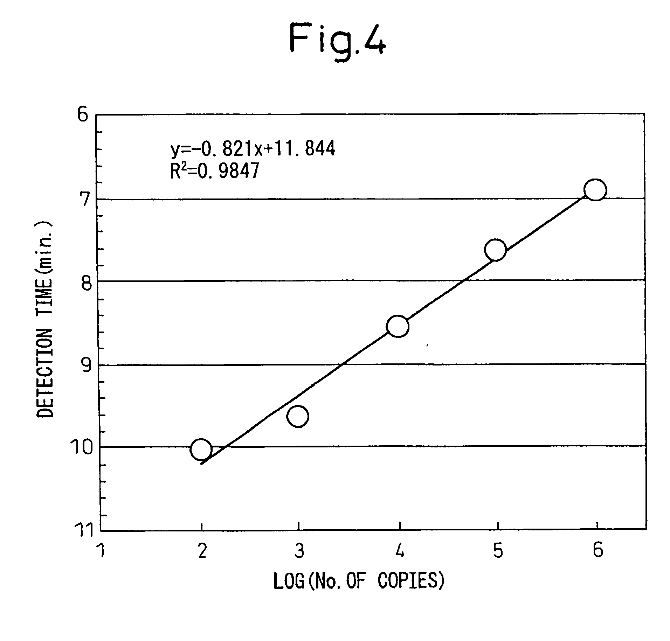

Fig. 4 shows a calibration curve obtained from the results of Fig. 3. The detection time was plotted against the logarithm of the initial number of copies of standard RNA, where the detection time was the time at which the fluorescent intensity ratio reached 1.2 in the results shown in Fig. 3. The formula inside the graph represents the primary regression and correlation function. The initial number of copies of an unknown sample is calculated based on this calibration curve and the detection time of the unknown sample.

### Best Mode for Carrying Out the Invention

The present invention will now be explained in greater detail.

The present invention provides a method for assaying stanniocalcin-1 (STC1) mRNA present in a sample, which method comprises a step of producing double-stranded DNA which contains a promoter sequence and a specific base sequence within the mRNA downstream from the promoter sequence, using a first primer which is homologous to at least one section downstream from the 5' end of the specific base sequence and a second primer which is complementary to at least one section upstream from the 3' end of the specific base sequence, wherein the promoter sequence is at the 5' end of either or both the first and second primers, a step of producing RNA transcript using the double-stranded DNA as template, a step of using the RNA transcript as template for subsequent DNA synthesis to produce the double-stranded DNA, a nucleic acid amplification step in which each of the aforementioned steps is repeated under conditions which promote each of the aforementioned steps simultaneously, and a step of measuring the amount of RNA transcript.

The "sample" according to this invention is nucleic acid extracted from a specimen such as blood, serum, plasma, tissue or other body fluid by a known method.

The "specific base sequence" according to the invention is the sequence of at least a portion of stanniocalcin-1 mRNA or a complementary sequence thereto, and it has a sequence in the region defined by the first and second primers. According to the invention, an RNA transcript derived from the specific base sequence is amplified. When the promoter sequence is added to the first primer, the STC1 mRNA is preferably cleaved at the 5' end of the specific base sequence before serving as the template for cDNA synthesis. The cleavage method is not particularly restricted, but a method is preferred in which an enzyme with ribonuclease H (RNaseH) activity is used to cleave the RNA portion of the RNA-DNA hybrid formed by adding an oligonucleotide with a sequence complementary to the region adjacent to and overlapping the 5' end of the specific base sequence of STC1 mRNA (cleavage oligonucleotide), and preferably the 3' end-OH of the cleavage oligonucleotide is appropriately modified, for example, aminated, to prevent extension reaction.

"Target nucleic acid" according to the invention refers to the region of the specific base sequence which is not homologous or complementary to the first and second primers, and it has a sequence capable of complementarily binding with a fluorescent intercalator dye-labeled nucleic acid probe. The fluorescent intercalator dye-labeled nucleic acid probe is therefore a sequence complementary to a portion of the specific base sequence according to the invention. Thus, as an example of one mode of the invention, if the specific base sequence is a sequence homologous to STC1 mRNA, the fluorescent intercalator dye-labeled nucleic acid probe may have a sequence comprising at least 15 contiguous bases of the sequence listed as SEQ ID NO: 3, or if the specific base sequence is a sequence complementary to STC1 mRNA, the fluorescent intercalator dye-labeled nucleic acid probe may have a sequence comprising at least 15 contiguous bases of the sequence complementary to the sequence listed as SEQ ID NO: 3. Since the target nucleic acid which is capable of complementarily binding with the fluorescent intercalator dye-labeled nucleic acid probe is a region which is neither homologous nor complementary to the first and second primers, the fluorescent intercalator dye-labeled nucleic acid probe does not undergo change in its fluorescent property by complementary binding with the first and second primers.

Either or both of the first and second primers of the invention have the promoter sequence at the 5' end, and the first primer is an oligonucleotide sufficiently complementary to the sequence complementary to STC1 mRNA while the second primer is an oligonucleotide sufficiently complementary to STC1 mRNA. Here, "sufficiently complementary" means that it is capable of complementarily binding with the specific base sequence or with the sequence complementary to that sequence under a stringent condition, such as the reaction conditions (reaction temperature and composition of salts, etc.) in the nucleic acid amplification step of the invention, e.g. those specified in the Examples shown below.

In order for the first primer to be sufficiently complementary to the sequence complementary to STC1 mRNA, the second primer to be sufficiently complementary to STC1 mRNA and the fluorescent intercalator dye-labeled nucleic acid probe to be sufficiently complementary to the target nucleic acid, preferably the first primer comprises at least 15 contiguous bases of the sequence listed as SEQ ID NO: 1, the second primer comprises at least 15 contiguous bases of the sequence listed as SEQ ID NO: 2, and the fluorescent intercalator dye-labeled nucleic acid probe comprises at least 15 contiguous bases of the sequence listed as SEQ ID NO: 3 or the sequence complementary to that sequence, and more preferably a combinations of the first primer, second primer and fluorescent intercalator dye-labeled nucleic acid probe are used.

The first primer, second primer and fluorescent intercalator dye-labeled nucleic acid probe may be oligonucleotides of any 15 or more contiguous bases within the range of SEQ ID NO: 1, SEQ ID NO: 2 and SEQ ID NO: 3, respectively. There is no particular restriction on the oligonucleotide lengths, but they are preferably 15-35 bases. While it is necessary to appropriately set the lengths in consideration of the Tm value, etc., the function and effect of the invention are the same regardless of the selected oligonucleotides so long as they are within the range of SEQ ID NO: 1, SEQ ID NO: 2 and SEQ ID NO: 3, respectively.

The "promoter sequence" according to the invention is a sequence which binds RNA polymerase to initiate transcription, and certain sequences are known corresponding to different variants of RNA polymerase. The RNA polymerase used is not particularly restricted; commonly employed T7 phase RNA polymerase, T3 phage RNA polymerase or SP6 phage RNA polymerase are suitable, and their corresponding promoter sequences may be used.

Various enzymes are required for the STC1 mRNA assay method of the invention (an enzyme with RNA-dependent DNA polymerase activity which uses single-stranded RNA as template (reverse transcriptase), an enzyme with RNaseH activity, an enzyme with DNA-dependent DNA polymerase activity which uses single-stranded DNA as template, and an enzyme with RNA polymerase activity). Each of these enzymes may be an enzyme having several activities, or multiple enzymes having each respective activity may be used. Also, for example, an enzyme with RNA polymerase activity may be added to a reverse transcriptase having RNA-dependent DNA polymerase activity which uses single-stranded RNA as template, RNaseH activity and DNA-dependent DNA polymerase activity which uses single-stranded DNA as template, or if necessary an enzyme with RNaseH activity may be further added as a supplement. Preferred reverse transcriptases of such a type which are frequently used include AMV reverse transcriptase, M-MLV reverse transcriptase, and their derivatives.

When reverse transcription is carried out in the presence of the aforementioned first and second primers (where the 5' end of the first or second primer contains the promoter sequence), the second primer binds to the specific sequence within STC1 mRNA and cDNA synthesis is accomplished by an enzyme having RNA-dependent DNA polymerase activity. The RNA portion of the obtained RNA-DNA hybrid is degraded by an enzyme with RNaseH activity, and dissociates to allow binding of the first primer to the cDNA. Here, since the first and second primers cannot bind to double-stranded DNA under the conditions described by the present invention, the promoter sequence is not added to double-stranded DNA such as chromosomal DNA, for example.

Next, double-stranded DNA derived from the specific base sequence and having the promoter sequence at the 5' end is produced using an enzyme with DNA-dependent DNA polymerase activity. This double-stranded DNA contains the specific base sequence downstream from the promoter sequence, and an RNA transcript derived from the specific base sequence is produced using an enzyme with RNA polymerase activity. The RNA transcript serves as the template for double-stranded DNA synthesis using the first and second primers, and the series of reactions proceeds as a chain-reaction resulting in amplification of the RNA transcript.

Needless to mention, the known components necessary for each of the enzymes used to conduct the chain-reaction include at least a buffer, magnesium salts, potassium salts, nucleoside triphosphates and ribonucleoside triphosphates. As additives to adjust the reaction efficiency there may be included dimethylsulfoxide (DMSO), dithiothreitol (DTT), bovine serum albumin (BSA), glucose and the like.

For example, when AMV transcriptase and T7 RNA polymerase are used, the reaction temperature is preferably in the range of 35-65°C and more preferably in the range of 40-45°C. The RNA amplification stage proceeds at a constant temperature, wherein the reaction temperature may be set to any temperature at which the transcriptase and RNA polymerase exhibit their activity.

The amount of amplified RNA transcript may be assayed by a known nucleic acid assay method. The assay method utilized may be a method employing electrophoresis or liquid chromatography, or a hybridization method using a nucleic acid probe labeled with a detectable label (for example, a dye or enzyme). However, these are multistep procedures requiring BF separation, and because the amplified product is removed out of the system for analysis, secondary contamination is a risk by escape of the amplified product into the environment. In order to overcome these problems, it is preferred to use a nucleic acid probe designed so that the fluorescent property changes by complementary binding with the target nucleic acid. As a more suitable method, there may be mentioned a method in which the nucleic acid amplification step is carried out in the presence of a nucleic acid probe which is labeled with a fluorescent intercalator dye and which is designed so that formation of a complementary double strand with the target nucleic acid causes the fluorescent property to change due to intercalation of the fluorescent intercalator dye portion in the complementary double strand portion, and this change in fluorescent property is measured (see Japanese Unexamined Patent Publication No. 2000-14400 and Ishiguro, T. et al, (2003) Anal. Biochem., 314, 77-86).

There are no particular restrictions on the fluorescent intercalator dye, and commonly employed oxazole yellow, thiazole orange, ethidium bromide and their derivatives may be used. The change in fluorescent property may be a change in fluorescent intensity. In the case of oxazole yellow, for example, it is known that intercalation into double-stranded DNA significantly increases the fluorescence at 510 nm (excitation wavelength: 490 nm). The fluorescent intercalator dye-labeled nucleic acid probe is an oligonucleotide sufficiently complementary to the RNA transcript, having a structure with the fluorescent intercalator dye bonded to the end or to a phosphate diester or base portion via an appropriate linker, and being appropriately modified at the 3' end-OH to prevent extension at the 3' end-OH (see Japanese Unexamined Patent Publication HEI No. 8-211050 and Ishiguro, T. et al, (1996) Nucleic Acids Research., 24, 4992-4997).

The labeling of the fluorescent intercalator dye on the oligonucleotide may be accomplished by introducing a functional group into the oligonucleotide by a known method for bonding of the fluorescent intercalator dye (see Japanese Unexamined Patent Publication No. 2001-13147 and Ishiguro, T. et al, (1996) Nucleic Acids Research., 24, 4992-4997). The method used to introduce the functional group may employ the conventionally used Label-ON Reagents (Clontech).

According to one mode of the invention, an amplification reagent, comprising at least a first primer with the T7 promoter sequence at the 5' end (including 15 contiguous bases of the sequence listed as SEQ ID NO: 1), a second primer (including 15 contiguous bases of the sequence listed as SEQ ID NO: 2), a fluorescent intercalator dye-labeled nucleic acid probe (including 15 contiguous bases of the sequence listed as SEQ ID NO: 3), a cleavage oligonucleotide (including 15 contiguous bases of the sequence listed as SEQ ID NO: 13, and having a sequence complementary to the region adjacent to and overlapping the 5' end of the specific base sequence), AMV reverse transcriptase, T7 RNA polymerase, buffer, magnesium salt, potassium salt, nucleoside triphosphates, ribonucleoside triphosphates and dimethylsulfoxide (DMSO), is added to a sample, and reaction is carried out isothermally at a reaction temperature of 35-65°C (preferably 40-45°C) while periodically measuring the fluorescent intensity of the reaction solution.

For this mode, the amplification curve of fluorescent intensity indicates the time shift in direct proportion to the logarithm of the initial RNA amount, and therefore the initial RNA amount in an unknown sample can be quantified by drawing a time-indicating calibration curve based on a known concentration of standard RNA. Also, since the fluorescent intensity is periodically measured, the measurement may be terminated at any time in which a significant increase in fluorescence is observed, and normally the nucleic acid amplification and assay can be completed with results within an hour, or within 30 minutes if the system is optimized.

Of special note is that all of the materials in the measuring reagent may be sealed in a single vessel. By carrying out the procedure of dispensing a fixed amount of sample in such a single vessel, it is possible to amplify and detect STC1 mRNA automatically at a later time. The vessel needs to have at least a portion thereof constructed of a transparent material in order to allow external measurement of the signal emitted by the fluorescent dye, and from the standpoint of preventing contamination it is preferably one which can be sealed after dispensing of the sample.

Since the RNA amplification and assay method of the mode described above may be carried out in a single stage and at constant temperature, the method is more convenient and suitable for automation than RT-PCR. However, because the reaction is carried out at a relatively low constant temperature of 35-65°C without the denaturation and annealing of RT-PCR, it is more prone to effects of non-specific amplification products such as primer dimers and the higher-order structure of the target RNA, such that a very elaborate design is necessary for construction of the assay system, as compared to RT-PCR, and for this reason it had not been previously possible to realize STC1 mRNA assay in a rapid, convenient, isothermal and single-stage manner using such an RNA amplification/assay method. According to the invention it has become possible for the first time to achieve highly specific, highly sensitive, rapid, convenient, isothermal, single-stage assay of STC1 mRNA.

The present invention may be applied as an indicator for early detection of leukemia, breast cancer and other cancers, monitoring of the effects of treatment such as chemotherapy, detection of microresidual lesions, prognosis and treatment protocol decision-making. Needless to mention, it may also be applied for the purpose of research, diagnosis and monitoring of disease progression in relation to any disease in which STC1 is implicated, such as Werner syndrome and other hereditary conditions, bone metabolic disorders, cerebral infarction and the like.

According to the present invention it has become possible to carry out isothermal, single-stage, convenient, rapid and highly sensitive assay of STC1 mRNA. The invention may therefore be applied for early detection of leukemia, breast cancer and other cancers, monitoring of the effects of treatment such as chemotherapy, detection of microresidual lesions and prognosis, and as an indicator for treatment protocol decision-making. Since the invention can be carried out in a single stage and in a sealed vessel, it is possible to minimize the risk of environmental contamination by secondary contaminating amplification products. Moreover since the method is conducted in a single stage in a convenient and rapid manner, multiple specimens can be processed even manually, and it is possible to minimize procedural complexity which leads to poor reproducibility. In addition, the RNA amplification method of the invention amplifies only RNA, and therefore it is possible to amplify and assay mRNA in a stringent manner without a step for complete removal of double-stranded DNA, as in RT-PCR. In other words, the method of the invention is suitable for highly sensitive and rapid analysis of expression. Furthermore, since the reaction can be carried out from start to finish at a constant temperature in a single stage, there is no need for a thermal cycling mechanism as in PCR, and automation is thus facilitated.

### Examples

The present invention will now be explained in greater detail with the understanding that the invention is not limited to the examples.

### Example 1

STC1 RNA was transcribed in vivo using as template double-stranded DNA containing STC1 cDNA (comprising bases 242-1028, with base numbering according to National Center Biotechnology Information Accession No. BC029244) downstream from the SP6 phage RNA polymerase promoter, and this was followed by treatment with DNaseI for complete digestion of the double-stranded DNA, and then purification and preparation of the RNA. The RNA was quantified by measurement of the absorbance at 260 nm.

Although this RNA is the subject of the assay in the following examples, they may be equally applied to assay of STC1 mRNA, which is the subject of assay according to the invention.

### Example 2

A fluorescent intercalator dye-labeled oligonucleotide probe was prepared. Label-ON Reagents (Clontech) was used to introduce an amino group at the 13th base from the 5' end of the sequences listed as SEQ ID NO: 10 and 11 (C in SEQ ID NO: 10 and T in SEQ ID NO: 11), and the 3' end was also modified with biotin. Oxazole yellow was bonded to the amino group by the method described in Ishiguro, T. et al (1996) (Fig. 1B). Oxazole yellow was also bonded via a linker at the phosphate diester bond between the 10th base (C) and the 11th base (A) from the 5' end of the sequence listed as SEQ ID NO: 11, by the method described in Ishiguro, T. et al (1996) to prepare an oxazole yellow-labeled nucleic acid probe (Fig. 1A).

### Example 3

STC1 RNA in various initial numbers of copies were detected using the method of the present invention.
(1) STC1 RNA (comprising bases 242-1028) was diluted to 50, 100 and 1000 copies/5 µl using an RNA diluent (10 mM Tris·HCl (pH 8.0), 1 mM EDTA, 0.25 U/µl ribonuclease inhibitor, 5 mM DTT) for use as RNA samples. The RNA diluent was used as the negative standard (0 copies).
(2) A 20 µl portion of a reaction mixture having the composition shown below was dispensed into a 0.5 ml volume PCR tube (Gene Amp Thin-Walled Reaction Tubes, Perkin-Elmer), and 5 µl of RNA sample was added. Reaction mixture composition: The concentrations are the final concentrations (in 30 µl) after addition of the enzyme solution
   60 mM Tris-HCl (pH8.6)
   17 mM magnesium chloride
   100 mM potassium chloride
   1 mM DTT
   0.25 mM each of dATP, dCTP, dGTP, dTTP
   3 mM each of ATP, CTP, UTP
   2.25 mM GTP
   3.6 mM ITP
   1 µM first primer (SEQ ID NO: 5): The first primer comprised the T7 RNA polymerase promoter sequence (SEQ ID NO: 17) added to the 5' end of the base sequence of SEQ ID NO: 5.
   1 µM second primer (SEQ ID NO: 8)
   25 nM fluorescent intercalator dye-labeled nucleic acid probe (SEQ ID NO: 11): The nucleic acid probe was prepared using Label-ON Reagents in Example 2.
   0.16 µM cleavage oligonucleotide (SEQ ID NO: 15): The 3' end-OH of the oligonucleotide was modified with an amino group.
   6 U/30 µl ribonuclease inhibitor (Takara Bio) 13% DMSO
(3) The reaction mixture was incubated at 43°C for 5 minutes, and then 5 µl of enzyme solution having the following composition and preheated at 43°C for 2 minutes was added. Enzyme solution composition: Final concentrations (in 30 µl) during reaction
   2% sorbitol
   8 U/30 µl AMV reverse transcriptase (Takara Bio)
   142 U/30 µl T7 RNA polymerase (GIBCO)
   3.6 µg/30 µl bovine serum albumin
(4) Reaction was then carried out at 43°C while periodically measuring the fluorescent intensity of the reaction mixture (excitation wavelength: 470 nm, fluorescent wavelength: 520 nm) using a fluorescence spectrometer equipped with a heat regulating function, capable of direct measurement of the PCR tube. Fig. 2 shows the time-dependent change in fluorescent intensity ratio of the reaction mixture (fluorescent intensity at prescribed time divided by background fluorescent intensity), where the enzyme addition point was marked as 0 min. The background fluorescent intensity was defined as the average value of the fluorescent intensity at 90, 120 and 150 seconds after start of the assay for each sample. The results in Fig. 2 show a fluorescence profile dependent on the initial concentration of STC1 RNA, wherein an increase in fluorescence is seen after about 12 minutes with 50 copies/test of STC1 RNA. It was thus demonstrated that the method of the invention allows highly sensitive and rapid quantification of STC1 RNA.

### Example 4

STC1 RNA was assayed by the method of the invention using various combinations of the first primer, second primer, fluorescent intercalator dye-labeled nucleic acid probe and cleavage oligonucleotide.
(1) STC1 RNA was diluted to 10³ copies/5 µl using an RNA diluent (10 mM Tris·HCl (pH 8.0), 1 mM EDTA, 0.25 U/µl ribonuclease inhibitor, 5.0 mM DTT) for use as RNA samples.
(2) A 20 µl portion of a reaction mixture having the composition shown below was dispensed into a 0.5 ml volume PCR tube (Gene Amp Thin-Walled Reaction Tubes, Perkin-Elmer), and 5 µl of RNA sample was added. Reaction mixture composition: The concentrations are the final concentrations (in 30 µl) after addition of the enzyme solution
   60 mM Tris·HCl (pH8.6)
   17 mM magnesium chloride
   100 mM potassium chloride
   1 mM DTT
   0.25 mM each of dATP, dCTP, dGTP, dTTP
   3 mM each of ATP, CTP, UTP
   2.25 mM GTP
   3.6 mM ITP
   1 µM first primer (SEQ ID NO. shown in Table 1): The first primer comprised the T7 RNA polymerase promoter sequence (SEQ ID NO: 17) added to the 5' end of the base sequence of the corresponding SEQ ID listed in Table 1.
   1 µM second primer (SEQ ID NO. shown in Table 1)
   25 nM fluorescent intercalator dye-labeled nucleic acid probe (SEQ ID NO. shown in Table 1): The nucleic acid probe was prepared according to Example 2.
   0.16 µM cleavage oligonucleotide (SEQ ID NO. shown in Table 1): The 3' end-OH of the oligonucleotide was modified with an amino group.
   6 U/30 µl ribonuclease inhibitor (Takara Bio) 13% DMSO
(3) The reaction mixture was incubated at 43°C for 5 minutes, and then 5 µl of enzyme solution having the following composition and preheated at 43°C for 2 minutes was added. Enzyme solution composition: Final concentrations (in 30 µl) during reaction
   2% sorbitol
   8 U/30 µl AMV reverse transcriptase (Takara Bio)
   142 U/30 µl T7 RNA polymerase (GIBCO)
   3.6 µg/30 µl bovine serum albumin
(4) Reaction was then carried out at 43°C while periodically measuring the fluorescent intensity of the reaction mixture (excitation wavelength: 470 nm, fluorescent wavelength: 520 nm) using a fluorescence spectrometer equipped with a heat regulating function, capable of direct measurement of the PCR tube. Table 1 shows the results, where (+) was assigned if the fluorescent intensity ratio of the reaction mixture (fluorescent intensity at prescribed time divided by background fluorescent intensity) exceeded 1.2, and that time was recorded as the detection time with the enzyme addition point marked as 0 min.

When the combinations of first primer, second primer, fluorescent intercalator dye-labeled nucleic acid probe and cleavage oligonucleotide listed in Table 1 were used, 10³ copies/test of STC1 RNA was detected within 20 minutes with all of the combinations. Specifically, when using a combination consisting of a sequence selected from SEQ ID NO: 4-6 as the first primer, a sequence selected from SEQ ID NO: 7-9 as the second primer, a sequence selected from SEQ ID NO: 10-11 as the fluorescent intercalator dye-labeled nucleic acid probe and a sequence selected from SEQ ID NO: 14-16 as the cleavage oligonucleotide, all of the STC1 RNA samples were rapidly detectable. SEQ ID NO: 4-6 are all partial sequences of SEQ ID NO: 1, SEQ ID NO: 7-9 are all partial sequences of SEQ ID NO: 2, SEQ ID NO: 10-11 are both partial sequences of SEQ ID NO: 3 and SEQ ID NO: 14-16 are all partial sequences of SEQ ID NO: 13. Thus, it was demonstrated that oligonucleotides falling in the ranges of the sequences listed as SEQ ID NO: 1, SEQ ID NO: 2, SEQ ID NO: 3 and SEQ ID NO: 13 all have the same function and effect according to the invention. This indicates that STC1 RNA can be rapidly detected by an RNA amplification and assay method employing the first primer, second primer and fluorescent intercalator dye-labeled nucleic acid probe described by the present invention.

**Table 1**

| Oligonucleotide combination | | | | Measurement results | |
|---|---|---|---|---|---|
| First primer (SEQ ID NO.) | Second primer (SEQ ID NO.) | Fluorescent intercalator dye-labeled nucleic acid probe (SEQ ID NO.) | Cleavage oligonucleotide (SEQ ID NO.) | Detection time (min) | Judgment |
| 4 | 7 | 10 | 14 | 10.8 | + |
| 4 | 8 | 10 | 14 | 13.5 | + |
| 4 | 8 | 11 | 14 | 13.5 | + |
| 4 | 9 | 10 | 14 | 14.6 | + |
| 4 | 9 | 11 | 14 | 14.6 | + |
| 5 | 7 | 10 | 15 | 10.5 | + |
| 5 | 8 | 10 | 15 | 11.1 | + |
| 5 | 8 | 11 | 15 | 11.2 | + |
| 5 | 9 | 10 | 15 | 12.5 | + |
| 5 | 9 | 11 | 15 | 11.7 | + |
| 6 | 7 | 10 | 16 | 18.9 | + |
| 6 | 8 | 10 | 16 | 14.3 | + |
| 6 | 8 | 11 | 16 | 13.6 | + |
| 6 | 9 | 10 | 16 | 18.2 | + |
| 6 | 9 | 11 | 16 | 14.4 | + |

The combinations of oligonucleotides in the above table were used for RNA amplification/fluorescent assay with 10³ copies/test of STC1 RNA as the sample. The symbol (+) was assigned if the fluorescent intensity ratio exceeded 1.2, and that time was recorded as the detection time.

### Example 5

STC1 RNA was quantified using the method of the present invention.
(1) The STC1 RNA was diluted to 10², 10³, 10⁴, 10⁵ and 10⁶ copies/5 µl using an RNA diluent (10 mM Tris·HCl (pH 8.0), 1 mM EDTA, 0.25 U/µl ribonuclease inhibitor, 5.0 mM DTT) for use as calibration curve standard RNA samples. The diluent was used as the negative standard (NEG). Samples A (10² copies/5 µl), B (10³ copies/5 µl), C (10⁴ copies/5 µl), D (10⁵ copies/5 µl) and E (10⁶ copies/5 µl) were prepared in the same manner.
(2) A 20 µl portion of a reaction mixture having the composition shown below was dispensed into a 0.5 ml volume PCR tube (Gene Amp Thin-Walled Reaction Tubes, Perkin-Elmer), and 5 µl each of the aforementioned standard RNA and sample were added.
   Reaction mixture composition: The concentrations are the final concentrations (in 30 µl) after addition of the enzyme solution
   60 mM Tris·HCl (pH8.6)
   17 mM magnesium chloride
   100 mM potassium chloride
   1 mM DTT
   0.25 mM each of dATP, dCTP, dGTP, dTTP
   3 mM each of ATP, CTP, UTP
   2.25 mM GTP
   3.6 mM ITP
   1 µM first primer (SEQ ID NO. 5): The first primer comprised the T7 RNA polymerase promoter sequence (SEQ ID NO: 17) added to the 5' end of the base sequence of SEQ ID NO: 5.
   1 µM second primer (SEQ ID NO. 8)
   25 nM fluorescent intercalator dye-labeled nucleic acid probe (SEQ ID NO. 12): The nucleic acid probe had oxazole yellow bonded via a linker at the phosphate diester, according to Example 2.
   0.16 µM cleavage oligonucleotide (SEQ ID NO. 15): The 3' end-OH of the oligonucleotide was modified with an amino group.
   6 U/30 µl ribonuclease inhibitor (Takara Bio) 13% DMSO
(3) The reaction mixture was incubated at 43°C for 5 minutes, and then 5 µl of enzyme solution having the following composition and preheated at 43°C for 2 minutes was added. Enzyme solution composition: Final concentrations (in 30 µl) during reaction
   2% sorbitol
   8 U/30 µl AMV reverse transcriptase (Takara Bio)
   142 U/30 µl T7 RNA polymerase (GIBCO)
   3.6 µg/30 µl bovine serum albumin
(4) Reaction was then carried out at 43°C while periodically measuring the fluorescent intensity of the reaction mixture (excitation wavelength: 470 nm, fluorescent wavelength: 520 nm) using a fluorescence spectrometer equipped with a heat regulating function, capable of direct measurement of the PCR tube. Fig. 3 shows the time-dependent change in fluorescent intensity ratio of the reaction mixture (fluorescent intensity at prescribed time divided by background fluorescent intensity), where the enzyme addition point was marked as 0 min.

Fig. 4 shows a calibration curve drawn from the detection time and the logarithm of the initial number of copies, where the detection time was the time at which the fluorescent intensity ratio reached 1.2 in the results shown in Fig. 3. Table 2 shows the results of quantifying the number of copies of sample from the calibration curve and detection time for each sample A, B, C, D and E.

As shown in Fig. 3, 10² copies/test was detected in approximately 10 minutes, and satisfactorily linearity was obtained between the detection time and the logarithm of the initial number of copies (Fig. 4). For the results in Table 2, the quantification results of samples A, B, C, D and E were values corresponding to the amounts of STC1 RNA addition. It was thus demonstrated that the method of the invention allows rapid, highly sensitive and specific quantification of STC1 RNA.

**Table 2**

| Sample | Calculated Copies/Test | Measured Copies/Test |
|---|---|---|
| A | 1 × 10² | 1.6 × 10² 1.5 × 10² |
| B | 1 × 10³ | 5.4 × 10² 6.6 × 10² |
| C | 1 × 10⁴ | 1.2 × 10⁴ 1.3 × 10⁴ |
| D | 1 × 10⁵ | 1.6 × 10⁵ 1.0 × 10⁵ |
| E | 1 × 10⁶ | 1.3 × 10⁶ |

Results for number of copies were calculated from detection times obtained from the fluorescence profile upon measurement of samples A (10² copies/5 µl), B (10³ copies/5 µl), C (10⁴ copies/5 µl), D (10⁵ copies/5 µl) and E (10⁶ copies/5 µl), and the calibration curve of Fig. 4.

## Claims

1. A method for assaying stanniocalcin-1 mRNA present in a sample, which method comprises
(1) a step of producing double-stranded DNA which contains a promoter sequence and a specific base sequence within said mRNA downstream from said promoter sequence, using a first primer which is homologous to at least one section downstream from the 5' end of said specific base sequence and a second primer which is complementary to at least one section upstream from the 3' end of said specific base sequence, wherein said promoter sequence is at the 5' end of either or both said first and second primers,
(2) a step of producing RNA transcript using said double-stranded DNA as template,
(3) a step of amplifying said RNA transcript in a chain-reaction manner using said RNA transcript as template for subsequent DNA synthesis, and
(4) a step of measuring the amount of said RNA transcript.

2. The method for assaying stanniocalcin-1 mRNA according to claim 1, **characterized by** using a combination of said first and second primers, wherein said first primer comprises at least 15 contiguous bases of the sequence listed as SEQ ID NO: 1, and said second primer comprises at least 15 contiguous bases of the sequence listed as SEQ ID NO: 2.

3. The method for assaying stanniocalcin-1 mRNA according to claim 1 or 2, **characterized in that** the assay of the amount of said RNA transcript is carried out by measuring the change in the fluorescent property of a nucleic acid probe which is labeled with a fluorescent intercalator dye and designed so that formation of a complementary double-stranded chain with the target nucleic acid causes the fluorescent intercalator dye portion to intercalate in said complementary double-stranded portion producing a change in its fluorescent property.

4. The method for assaying stanniocalcin-1 mRNA according to claim 3, **characterized by** using a combination of oligonucleotides consisting of said first primer comprising at least 15 contiguous bases of the sequence listed as SEQ ID NO: 1, said second primer comprising at least 15 contiguous bases of the sequence listed as SEQ ID NO: 2, and said fluorescent intercalator dye-labeled nucleic acid probe comprising at least 15 contiguous bases of the sequence listed as SEQ ID NO: 3 or the sequence complementary to said sequence.

5. An assay reagent for stanniocalcin-1 mRNA, **characterized by** containing as constituent elements oligonucleotides which have a combination of sequences consisting of a first primer comprising at least 15 contiguous bases of the sequence listed as SEQ ID NO: 1, and a second primer comprising at least 15 contiguous bases of the sequence listed as SEQ ID NO: 2, wherein a promoter sequence is at the 5' end of either or both of said first and second primers.

6. An assay reagent for stanniocalcin-1 mRNA, **characterized by** containing as constituent elements oligonucleotides which are a combination of sequences consisting of a first primer comprising at least 15 contiguous bases of the sequence listed as SEQ ID NO: 1, a second primer comprising at least 15 contiguous bases of the sequence listed as SEQ ID NO: 2, wherein a promoter sequence is at the 5' end of either or both of said first and second primers, and a fluorescent intercalator dye-labeled nucleic acid probe comprising at least 15 contiguous bases of the sequence listed as SEQ ID NO: 3 or the sequence complementary to said sequence.

7. Stanniocalcin-1 mRNA or a specific oligonucleotide which is complementary thereto, **characterized by** comprising at least 15 contiguous bases of the sequence listed as any one of SEQ ID NO: 1 to 3, or the sequences complementary to said sequences.
